# EUROPÄISCHE PATENTSCHRIFT

(11) **EP 2 957 220 B1**
(45) Veröffentlichungstag und Bekanntmachung des Hinweises auf die Patenterteilung: **24.04.2019**
(21) Anmeldenummer: 14172523.4
(22) Anmeldetag: 16.06.2014
(51) Int. Cl.: A61B 5/00, A61B 6/03, A61B 5/05

(54) **Computertomographie mit simultanem Magnetic Particle Imaging**
Computed tomography and simultaneous magnetic particle imaging
Enregistrement simultané d'imagerie par CT et d'imagerie de particules magnétiques

(43) Veröffentlichungstag der Anmeldung: 23.12.2015
(73) Patentinhaber: Universität zu Lübeck, 23562 Lübeck (DE)
(72) Erfinder: Kaethner, Christian, 23552 Lübeck (DE); Buzug, Thorsten, 23627 Groß Sarau (DE); Bringout, Gael, 23552 Lübeck (DE)
(74) Vertreter: RCD Patent Giesen, Schmelcher & Griebel Patentwanwälte PartG mbB

(56) Entgegenhaltungen:
- WO-A1-2013/088413
- US-A1- 2007 232 899

## Beschreibung

Die Erfindung betrifft einen Hybridscanner zur medizinischen Bildgebung eines Patienten, dessen Körperinneres eine Verteilung vorverabreichter, magnetisierbarer Partikel aufweist. Die Erfindung betrifft insbesondere eine Kombination aus Magnetic Particle Imaging und Röntgen-Computertomographie zur automatischen Erzeugung tomographischer Darstellungen des Patienten.

Die tomographische Bildgebung ist heute fester Bestandteil der klinischen Diagnostik. Die verwendeten Modalitäten lassen sich hierbei generell in zwei Kategorien einteilen: die morphologische Bildgebung, zu welcher die Computertomographie - CT - und die Magnetresonanztomographie - MRT - gehören, und die funktionelle Bildgebung, zu welcher Positronen-Emissions-Tomographie - PET -, Single-Photon-Emission-Computed-Tomography - SPECT - oder auch Magnetic Particle Imaging - MPI - zählen.

Fusioniert man die Bilddaten von beispielsweise MRT und PET, erhält man eine präzise Abbildung morphologischer Strukturen in Kombination mit den korrelierenden physiologischen Prozessen. Beispielsweise in der Arbeit von Wang et al. (2012), "Towards Omni-Tomography-Grand Fusion of Multiple Modalities for Simultaneous Interior Tomography", PLoS ONE 7(6): e39700. doi:10.1371/journal.pone.0039700 werden multimodale Bildgebungsvorrichtungen, die auch als "hybride Scanner" bezeichnet werden, vorgestellt.

Die Problematik existierender hybrider Scanner, z.B. MRT/PET, PET/CT, SPECT/CT, ist neben den hohen Kosten eine unzureichende zeitliche sowie örtliche Auflösung der funktionellen Informationen. Außerdem ist bei PET und SPECT unvermeidlich, dass radioaktiver Kontrastmittel zum Einsatz kommen. Dies möchte man vermeiden, da neben der Strahlenbelastung der Patienten und des untersuchenden Personals auch die Kosten für den erforderlichen Strahlenschutz erheblich sind.

Beim Magnetic Particle Imaging (MPI) werden lokale Konzentrationen magnetisierbarer Nanopartikel in einer a priori unbekannten räumlichen Verteilung im Innern eines Patienten ermittelt. Diese Nanopartikel sind nicht radioaktiv, und das MPI-Messverfahren bedarf auch sonst keiner ionisierenden Strahlung. Beispielsweise können super-paramagnetische Eisenoxidpartikel durch ein mit vorbestimmter Frequenz periodisch veränderliches Anregungsmagnetfeld, engl. "drive field", periodisch magnetisiert werden, wobei die Magnetisierung der Partikel nichtlinear von der Magnetfeldstärke abhängt. Durch Erfassen und Analysieren des Zeitverhaltens der Partikelmagnetisierung im Verhältnis zur Charakteristik des Anregungsfeldes kann auf die Partikelkonzentration geschlossen werden. Zur Eingrenzung der MPI-Messung auf kleine Volumina des Patienten wird das Anregungsmagnetfeld mit einem zeitlich konstanten Selektionsmagnetfeld, engl. "selection field", überlagert. Das Selektionsmagnetfeld weist an wenigstens einem vorbestimmten Punkt der Untersuchungsregion eine Nullstelle auf. Ausgehend von diesem so genannten feldfreien Punkt, kurz: FFP, steigt das Selektionsmagnetfeld in alle Richtungen schnell an, so dass magnetisierbare Nanopartikel schon in geringem Abstand zum FFP in die magnetische Sättigung gelangen. Weit vom FFP entfernte Nanopartikel reagieren dann kaum noch auf das Anregungsmagnetfeld und leisten keinen signifikanten Beitrag zum detektierten Signal. Das MPI-Messsignal stammt vielmehr aus der lokalen Umgebung des FFP und gibt über die dort vorhandene, lokale Partikelkonzentration Auskunft.

Das Anregungsfeld ist zeitabhängig und wird von Elektromagneten erzeugt. Mit Hilfe des Anregungsmagnetfeldes wird der FFP im Innern des Objekts bewegt, wobei er einer als FFP-Trajektorie bezeichneten Kurve im Raum folgt. Die FFP-Trajektorie kann einen ein- oder dreidimensionalen Verlauf aufweisen, in vielen MPI-Aufbauten ist sie jedoch zweidimensional, d.h. der FFP wird in einer vorbestimmten Ebene bewegt, wie z.B. in der US 2012/153949 A1.

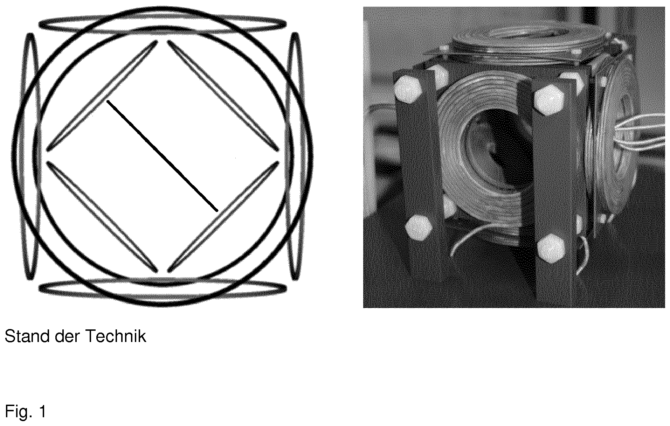

Alternativ kann statt eines einzelnen FFP auch eine feldfreie Linie, kurz: FFL, mit einer geeigneten Konfiguration magnetfelderzeugender Spulen generiert werden. Eine solche Konfiguration ist exemplarisch in Fig. 1 zu sehen, wobei im linken Bild die Spulenausrichtung schematisch zusammen mit der Ausrichtung der FFL in der Bildebene, xy-Ebene, unter einem Winkel gegen die x-Achse dargestellt ist. Die FFL kann durch die Wahl der Bestromung der Spulen modifiziert, insbesondere auch in der xy-Ebene parallelverschoben oder um das Zentrum rotiert, werden. Im rechten Bild von Fig. 1 ist eine Fotografie des Labormusters der Spulenanordnung zu sehen.

Die Verwendung einer FFL anstatt eines einzelnen FFP hat das Potenzial, die Datenakquisitionsdauer um eine Größenordnung zu verringern. Allerdings antworten alle magnetischen Partikel eines im Innern der Spulenanordnung platzierten Objekts entlang der FFL simultan auf eine Änderung des Anregungsfeldes, so dass die Auswertung der MPI-Messsignale im Hinblick auf lokale Konzentrationen, die man dann einzelnen Voxeln des Objekts zuweisen kann, erschwert wird.

Die MPI-Messergebnisse sind ortsabhängige Konzentrationen der magnetisierbaren Partikel, die wiederum bei geeigneter Auswahl und Vorverabreichung an den Patienten Informationen über die Gewebefunktionalität liefern können.

Demgegenüber erlaubt die Röntgen-CT die Bestimmung von ortsabhängigen Röntgenabschwächungskoeffizienten im Patientenkörper. Auf diese Weise wird die Gewebemorphologie erkennbar.

Beide Verfahren bieten ihre jeweiligen Vorteile. Die Verarbeitung der jeweiligen Datensätze zu für den behandelnden Arzt interpretierbaren Bildern stellt bekanntlich eine nicht zu unterschätzende Aufgabe dar.

Aus der Druckschrift WO 2013/088413 A1 gehen eine Vorrichtung und ein Verfahren zum Magnetic Particle Imaging hervor, bei denen zu einem bestimmten Zeitpunkt vor und/oder nach einer Messung ein Hintergrundsignal detektiert und in einem Nachbearbeitungsschritt vom Messsignal subtrahiert wird. Während der Messung des Hintergrundsignals befinden sich kein Objekt, kein Patient und keine MPIsensitive Probe im Messfeld des MPI-Gerätes. Für langsam veränderliche Störungen, die ein Hintergrundsignal hervorrufen, wird auch vorgeschlagen, das Hintergrundsignal zwischen den Messzeitpunkten, also während der eigentlichen MPI-Messung eines Objekts, zu interpolieren.

Die Druckschrift US 2007/0232899 A1 offenbart ein Verfahren zur Lokalisierung invasiver Instrumente in Echtzeit während eines medizinischen Eingriffs. Diese Arbeit bedient sich eines Messverfahrens, das dem Magnetic Particle Imaging (MPI) nachempfunden ist. Es findet jedoch keine Bildgebung im Sinne des MPI statt, sondern vielmehr wird eine "region of interest" (ROI) in kleine Subvolumina bzw. Voxel unterteilt und ermittelt, in welchem dieser Voxel sich das invasive Instrument gerade aufhält. Die magnetisierbaren Partikel sind dabei Bestandteile einer kolloidalen Suspension in einem Behälter, der beispielweise an der Spitze des Instruments angeordnet ist. Der Container mit der Suspension ist insoweit eine bauliche Komponente des invasiven Instruments und bewegt sich mit diesem beim Vorschub des Instruments. Beim üblichen MPI sind die Partikel funktionalisiert und werden durch physiologische Prozesse transportiert und im Gewebe verteilt. Das Resultat der Messung in der US 2007/0232899 A1 ist eine zeitabhängige Ortskoordinate bzw. ein Voxelindex, der allein der Beobachtung des Eingriffs dient und keine Rückschlüsse auf funktionale Eigenschaften des Gewebes zulässt. Verwendet wird diese Information zur Lokalisierung des Instruments und auch zum fortlaufenden Tracking, während das Instrument bewegt wird. Im Abs. 0019 der Druckschrift wird erläutert, dass es der Beschleunigung des Trackings dient, das regelmäßig zu wiederholende Selektieren des Testvolumens - jenes Voxels, der den FFP enthält - mit einem Durchlauf der Subvolumina in der Umgebung der zuletzt ermittelten Position des Instruments zu beginnen, da sich dieses stetig bewegen muss und man so frühzeitig fündig werden dürfte. Im Übrigen formuliert die Druckschrift in Abs. 0018 auch die Aufgabe, ihre MPI-Messdaten mit zeitgleich erzeugten Röntgenaufnahmen - ggf. aus verschiedenen Perspektiven erfasst - in einen gemeinsamen Bezugsrahmen zu setzen. Auf eine etwaige Störung der MPI-Messung durch den Röntgenapparat geht sie indes an keiner Stelle ein.

Ein medizinisches Bildgebungsverfahren eines Patienten, wobei MPI- und Röntgen-CT-Messwerte simultan und in Bezug zueinander erfasst und einer anschließenden gemeinsamen Auswertung zur Bildrekonstruktion zugeführt werden und bei dem das Körperinnere des Patienten eine Verteilung vorverabreichter, magnetisierbarer Partikel aufweist, umfasst die Schritte:
a. Durchstrahlen des Patienten mit Röntgenlicht unter Verdrehen der Strahlrichtung in einer Durchstrahlungsebene;
b. Erzeugen eines magnetischen Gradientenfeldes und zeitlich veränderlicher magnetischer Ansteuerungsfelder, die wenigstens einen feldfreien Punkt in der Durchstrahlungsebene generieren und entlang einer vorbestimmten Trajektorie bewegen;
c. Aufzeichnen der Röntgenbilder des Patienten zu einer Mehrzahl von vorbestimmten Strahlrichtungen unter Hinzufügen einer ersten Indexvariablen;
d. Erfassen der Antwort der magnetisierbaren Partikel auf die Bewegung des wenigstens einen feldfreien Punktes und Aufzeichnen der Messwerte unter Hinzufügen einer zweiten Indexvariablen;
e.Wiederholtes Messen des Magnetfeldes aus Schritt b. wenigstens an vorbestimmten Kontrollorten und Aufzeichnen der Messwerte unter Hinzufügen einer dritten Indexvariablen;
f. Zuführen der aufgezeichneten Messdaten zu einer gemeinsamen Auswertung zur Bildrekonstruktion unter Verwendung der vorbekannten Zuordnung der ersten, zweiten und dritten Indexvariablen zueinander.

2. Erfindungsgemäß wird hierzu ein geeigneter hybrider Scanner zur medizinischen Bildgebung gemäß Anspruch 1 vorgeschlagen.

Zur Klarstellung sei angemerkt, dass die Formulierung "wenigstens ein feldfreier Punkt" so zu verstehen ist, dass diese auch eine feldfreie Linie - aufgefasst als eine Aneinanderreihung einer Vielzahl nebeneinander existierender FFPs - mit umfasst.

Die Verfahrensschritte a. bis d. werden für eine vorbestimmte Zeitspanne, i. F. als ein Messdurchgang bezeichnet, simultan ausgeführt. Es liegt dabei auf der Hand, dass die erste und zweite Indexvariable im einfachsten Fall eine Zeitangabe sein können, beispielsweise die Anzeige der internen Uhr einer Einheit zur Datenaufzeichnung. Die erfindungsgemäßen Schritte e. und f. sind für den Fachmann unerwartet, da bei einer heute üblichen MPI-Messung gar kein Bedarf an einer Überprüfung des nach dem Biot-Savart-Gesetz bei bekannter Spulenanordnung und Spulenbestromung eindeutig vorhersagbaren Magnetfeldes besteht.

Ein solcher Bedarf entsteht aber bei der Kombination des MPI-Verfahrens mit einer unmittelbar benachbart angeordneten, zeitgleich aktiven CT-Gantry durch das Auftreten zusätzlicher elektrischer und magnetischer Felder aus dem Elektroantrieb der CT-Gantry, der Hochspannung-Röntgenquelle und aus den elektrischen Zuleitungen zu diesen.

Eine zweite Ursache für Störungen liegt in den unvermeidlichen Vibrationen der Magnetfeld erzeugenden Spulen, auf die wiederum Kräfte durch das Magnetfeld benachbarter Spulen wirken, welche dann zu Schwingungen im Takt vorbestimmter Stromflussänderungen anregen. In einem MPI-Scanner sind periodische Stromflussänderungen üblich, insbesondere zum repetierenden Bewegen eines FFP entlang einer Trajektorie.

Für einen hybriden MPI-CT-Scanner ist das Vibrationsproblem besonders deshalb zu beachten, weil eine zweckdienliche Vorrichtung eine deutlich größere Anzahl an Spulen als heute üblich aufweisen muss, deren Anordnung besonders präzise einzuhalten ist. Näheres wird weiter unten erläutert.

Eine dritte Ursache für Störungen des Magnetfeldes kann der lebende Patient sein, beispielsweise durch Eigenbewegungen.

Die Folge der vorgenannten Störungen kann insbesondere sein, dass der wenigstens eine feldfreie Punkt nicht genau dort vorliegt, wo er nach der Theorie eines alleinigen MPI-Verfahrens erzeugt werden müsste. Er kann verschoben vorliegen, wobei der Verschiebungsvektor zwischen Soll- und Ist-Position überdies im Verlauf der Messung veränderlich, mithin zeitabhängig, ist.

Für die angestrebte kombinierte Auswertung von CT- und MPI-Messwerten zur Bildrekonstruktion ist es deshalb erforderlich, sich Klarheit über die wahre Trajektorie des wenigstens einen FFP zu verschaffen und sich nicht allein auf seine theoretische Vorhersage zu verlassen.

Der Schritt e. sieht deshalb vor, das tatsächlich vom hybriden Scanner erzeugte Magnetfeld während eines Messdurchgangs wiederholt zu messen, um insbesondere die FFP-Verschiebung und ihre Zeitabhängigkeit zu erfassen.

Dabei reicht es aus, die Magnetfeldmessung an vorbestimmten Kontrollorten vorzunehmen, die vorzugsweise in oder nahe an der Durchstrahlungsebene liegen. Dem Fachmann wird die genaue Festlegung der Kontrollorte in Anbetracht des konkreten Aufbaus des hybriden Scanners im Einzelfall nicht schwerfallen.

Es ist eine mögliche Ausgestaltung an sich bekannte Magnetfelddetektoren an den vorbestimmten Kontrollorten anzuordnen und deren Messwerte auszulesen und unter Hinzufügen einer dritten Indexvariablen, beispielsweise auch hier einer Zeitangabe, aufzuzeichnen. In einer bevorzugten Ausgestaltung der Erfindung wird das Magnetfeld indes direkt mit Hilfe der Magnetfelderzeugerspulen detektiert, die auch zum Erfassen der Antwort der magnetisierbaren Partikel gemäß Schritt d. benutzt werden können. In diesem Fall sind die Kontrollorte womöglich nicht eindeutig benennbar, aber immer noch insofern vorbestimmt, als dass sie einer symmetrischen Verteilung um die Durchstrahlungsebene genügen, denn die Magnetfelderzeugerspulen sind entsprechend symmetrisch angeordnet.

Aus den Magnetfeldmesswerten, insbesondere den Magnetfeldamplituden, kann bei der späteren gemeinsamen Auswertung aller Messdaten auf eine etwaige Störung des Magnetfeldes während des Messdurchgangs geschlossen werden. Beispielsweise kann in Kenntnis der Koordinaten der Kontrollorte ein direkter Vergleich zwischen gemessener und theoretischer - d.h. ungestörter - Magnetfeldamplitude als Maß für eine Feldverzerrung ermittelt und auf eine Verschiebung eines FFP aus seiner theoretischen Trajektorie hochgerechnet werden. Alternativ können Feldverzerrungen auch aus Abweichungen einer gemessenen Amplitudenverteilung von einer theoretisch symmetrischen Verteilung bestimmt werden, ohne dass man die Koordinaten der Kontrollorte genau ermittelt, wenn man nur an der Berechnung einer Verschiebung eines FFP - einer Nullstelle des Feldes - aus der Durchstrahlungsebene - der theoretischen Symmetrieebene des Feldes - interessiert ist.

Mit Hilfe des berechneten, über einen Messdurchgang hinweg veränderlichen Verschiebungsvektors des wenigstens einen FFP können bei der gemeinsamen Auswertung aller Messdaten die tatsächlichen FFP-Trajektorien bestimmt und somit systematische Fehler vermieden werden.

Für den Fall, dass der Patient als Störquelle des Magnetfeldes ausgeschlossen werden kann, z.B. weil die Konstruktion des hybriden Scanners dies verhindert, kann der Schritt e. des Verfahrens vorteilhafterweise als einmaliger Vorversuch in Abwesenheit des Patienten ausgeführt werden. Praktisch kann eine Leermessung ohne Patient durchgeführt werden, die den späteren Messvorgang vollständig simuliert, d.h. insbesondere soll ein kompletter Messdurchgang unter Aktivierung der MPI-Spulen und des CT-Gantry einschließlich Röntgenlichtquelle erfolgen, und die Strahlrichtung und die Position des wenigstens einen FFP sollen genau wie in der späteren Messung vorgesehen verändert werden. Der alleinige Zweck dieser Leermessung ist das Erfassen und Aufzeichnen des Magnetfeldes.

Es ist hier hervorzuheben, dass Leermessungen bei üblichen Röntgen-CT- oder MPI-Messungen allenfalls zur Überprüfung der korrekten Gerätefunktion dienen und sehr selten sind. Für MPI-Scanner ist der Begriff Leermessung gar nicht ohne weiteres klar, denn im MPI-Messsignal wird der Frequenzbeitrag des Anregungsmagnetfeldes normalerweise unterdrückt. In Abwesenheit magnetisierbarer Partikel entsteht somit eigentlich auch kein verwertbares Signal. Wie bereits erläutert, steht die korrekte Erzeugung des Magnetfeldes beim MPI-Verfahren gewöhnlich nicht in Frage. Beim hybriden Scanner existiert aber eine ungewollte und doch schwer vermeidbare Wechselwirkung zwischen den im Übrigen völlig intakten Apparaten, die gesondert erfasst und berücksichtigt werden muss.

Die Aufzeichnung der Magnetfeldmesswerte in der Leermessung erfolgt auch unter Hinzufügen einer dritten Indexvariablen, die hier vorzugsweise die verstrichene Zeit seit Beginn des Messdurchgangs sein kann. In der späteren Messung mit Patient empfiehlt es sich, die erste und zweite Indexvariable genauso auf den Beginn des Messdurchgangs zu beziehen, um in der anschließenden gemeinsamen Auswertung von Röntgenbildern, MPI-Messdaten und Magnetfeldmessungen einen einfachen Bezug zueinander herzustellen.

Der in Schritt e. erzeugte Datensatz kann im günstigsten Fall als eine einmal zu erstellende, hiernach elektronisch zu speichernde und für jede Patientenmessung in Schritt f. aufzurufende Tabelle mit Kalibrierdaten sein. Ist der hybride Scanner allerdings eine mobile Einheit oder werden seine Komponenten, z.B. das Röntgen-CT-Gerät, gelegentlich auch separat benutzt und hierzu die MPI-Spulen vorübergehend entfernt, so ist Schritt e. mindestens nach jeder Neuaufstellung und Inbetriebnahme erneut durchzuführen.

Wenn der Patient überdies einen Störeinfluss haben könnte oder sich aus der anschließenden Bildrekonstruktion Anhaltspunkte ergeben, die auf einen Positionsfehler von wenigstens einem FFP hindeuten, dann sollte Schritt e. auch während der Patientenmessung ausgeführt werden. Dabei kann es von Vorteil sein, die Magnetfeldmesswerte mit und ohne Patient miteinander zu vergleichen und/oder die Messwertedifferenz unter Hinzufügen der dritten Indexvariablen aufzuzeichnen.

Für eine Variante des Verfahrens wird vorgeschlagen, die in den Schritten e. und f. bestimmten Verschiebungsvektoren des wenigstens einen FFP direkt während der Messung zu kompensieren, indem eine die Verschiebung ausgleichende Bestromung der Magnetfelderzeugerspulen durchgeführt wird. Dabei ist es besonders bevorzugt, dass die in Schritt e. erfassten Messwerte als zeitlich Schritt haltendes Feedback zur Steuerung der Magnetfelderzeugung in Schritt b. herangezogen werden. Es ist also vorgesehen, den voraussichtlich erst nach dem Ende des Messdurchgangs ausführbaren Schritt f. nicht zwingend abzuwarten, sondern eine Feldverzerrungskorrektur in Echtzeit vorzunehmen. In diesem Fall erübrigt sich eine Leermessung zur Kalibrierung wie oben beschrieben, aber Schritt e. ist bei jeder Patientenmessung notwendig. Im Prinzip erübrigt sich zwar auch das explizite Aufzeichnen des - nunmehr gegen Störeinflüsse kompensierten - Magnetfeldes für die nachfolgende Bildrekonstruktion, aber man wird schon zu Zwecken der Qualitätssicherung ein Protokoll des Magnetfeldes während der Messung aufbewahren wollen.

Während der Durchleuchtung eines Patienten wird die Röntgenstrahlrichtung programmgesteuert geändert, z.B. indem Röntgenquelle und Flächendetektor gemeinsam um den Patienten herum bewegt werden. Der wenigstens eine FFP wird gleichzeitig entlang vorbestimmter Trajektorien durch das Körperinnere des Patienten bewegt. Dabei ist es nun möglich, die FFP-Trajektorie und die Strahlrichtung in zeitlich Schritt haltender Weise aufeinander abzustimmen. Dies ist vorteilhaft, um entlang der Röntgenstrahlrichtung eine schnelle Lokalisierung von Zielstrukturen vorzunehmen, die mit MPI gut erkennbar sind, z.B. Gewebe mit hoher Partikelkonzentration. Diese zeitgleiche Lokalisierung entlang der Strahlrichtung kann der Formulierung einer zweckmäßigen Nebenbedingung für die spätere CT-Rekonstruktion dienen.

In einer Ausgestaltung des Verfahrensschritts b. weist die Trajektorie eines einzelnen FFP überwiegend Wegstreckenabschnitte entlang der Röntgenstrahlrichtung auf. Beispielsweise beschreibt die FFP-Trajektorie entlang einer Achse senkrecht zur Röntgenstrahlrichtung eine Rechteckfunktion mit zahlreichen hohen und schmalen Rechtecken. Die langen Rechteckkanten verlaufen dann in Strahlrichtung, und der FFP bewegt sich die meiste Zeit entlang dieser langen Kanten. Wird der Röntgenstrahl gedreht, dann kann auch ohne weiteres die Schritt haltende Drehung der Trajektorie vorgenommen werden.

In einer weiteren Ausgestaltung des Verfahrensschrittes b. wird eine feldfreie Linie in der Durchstrahlungsebene periodisch hin- und her bewegt ohne ihre Richtung zu ändern. Auf diese Weise überstreicht sie in kurzer Zeit das gesamte in der Durchstrahlungsebene befindliche Teilvolumen des Patienten. Die Richtungen von Röntgenstrahl und FFL werden nun unter einem vorbestimmten Winkel zueinander ausgerichtet. Beispielsweise können beide Richtungen übereinstimmen, d.h. der Winkel ist Null. Alternativ kann der Winkel auch 90° betragen, so dass die FFL senkrecht zur Röntgenstrahlrichtung orientiert ist und zugleich entlang der Strahlrichtung bewegt wird. Die Ausrichtung der FFL wird Schritt haltend angepasst, wenn sich die Einstrahlrichtung des Röntgenlichts ändert.

Die vorgenannten Varianten von Schritt b. sind nur mit einem hybriden MPI-CT-Scanner ausführbar und setzen daher auch die Schritte e. und f. voraus.

Die Erfindung wird nachfolgend näher erläutert anhand von Ausführungsbeispielen. Dabei zeigt:
Fig. 1 eine Spulenanordnung zur Erzeugung einer feldfreien Linie (FFL), links: Skizze mit FFL in der Bildebene, rechts: Fotografie einer realisierten Anordnung;
Fig. 2 Skizze einer FFP-erzeugenden Spulenanordnung mit Spalt in der xy-Ebene, oben: perspektivisch, unten: Aufsicht auf die yz-Ebene;
Fig. 3 Skizze einer FFL-erzeugenden Spulenanordnung mit Spalt in der xy-Ebene, oben: perspektivisch, unten: Aufsicht auf die yz-Ebene;

Ein erfindungsgemäßer hybrider MPI-CT-Scanner besteht beispielsweise aus einem kommerziell erhältlichen CT-Scanner-Gantry, also einer Röntgenquelle und einem Flächendetektor, die in einem ringförmigen Gehäuse einander gegenüber stehend angeordnet sind, so dass das Innere des Rings durchstrahlt und abgelichtet werden kann. Röntgenquelle und Detektor sind gewöhnlich innerhalb des Rings gemeinsam beweglich und können, z.B. mittels eines elektrischen Antriebs, um das Objekt im Ringinnern rotiert werden. Die Durchstrahlungsebene fällt mit der Ebene des Rings zusammen. Die erfindungsgemäße Vorrichtung weist überdies eine zur Ringebene symmetrische Anordnung von Magnetfelderzeugerspulen auf, die wenigstens einen FFP in der Ringebene erzeugen und unter Änderung der die Spulen durchfließenden Ströme bewegen können. Die Magnetfelderzeugerspulen und der CT-Scanner-Gantry werden jeweils durch eine eigene Ansteuereinrichtung angesteuert. Diese Ansteuereinrichtungen können in baulicher Einheit ausgeführt sein.

Bevorzugt ist eine Kommunikation zwischen der ersten Ansteuereinrichtung, die u.a. den elektrischen Antrieb des CT-Scanner-Gantry kontrolliert und dadurch die Röntgenstrahlrichtung steuert, und der zweiten Ansteuereinrichtung, die die Magnetfelderzeugerspulen bestromt, vorgesehen. Diese Kommunikation soll über eine rechnergestützte Auswerteeinheit, beispielsweise einen Personal Computer, erfolgen, der die Steuerparameter der beiden Ansteuereinrichtungen protokolliert. Als Steuerparameter kommen beispielsweise die Stromstärken der einzelnen Magnetfelderzeugerspulen und die Ansteuerdaten für den CT-Scanner-Gantry-Antrieb und für die Röntgenlichtquelle in Betracht. Alternative Steuerparameter, die als abgeleitete Daten auf den vorgenannten Rohdaten beruhen, kommen aber auch in Frage, z.B. Röntgenstrahlrichtungen und/oder die aktuellen Koordinaten des wenigstens einen FFP als Funktion der Zeit.

Es ist zu betonen, dass die Steuerparameter vorbekannt sind. Sie werden gewöhnlich programmgesteuert vorgegeben und nicht gemessen. Es liegt auf der Hand, diese Vorgaben auch für die spätere Auswertung zu protokollieren.

Die Auswerteeinheit soll darüber hinaus die MPI-Messwerte und die Röntgenbilder erfassen und unter Hinzufügen je einer Indexvariablen nicht-flüchtig abspeichern. Überdies erfasst und speichert sie die Magnetfeldmessdaten aus Erfindungsschritt e. und hält die Kalibriermessdaten aus zuvor durchgeführten Leermessungen elektronisch gespeichert vor.

Die Indexvariablen können Zeitangaben sein, z.B. bezogen auf den Beginn des Messdurchgangs. Die Indexvariablen können aber auch beliebig aus den protokollierten Steuerparametern ausgewählt sein, z.B. kann die erste Indexvariable eine Winkelangabe für die Röntgenstrahlrichtung, die zweite Indexvariable ein Stromvektor mit Einzelspulenströmen als Komponenten und die dritte Indexvariable eine Zeitangabe sein. Durch die Programmvorgabe der Steuerparameter bleibt die Möglichkeit der in Erfindungsschritt f. auszuführenden Zuordnung der Messdaten zueinander gewahrt.

Die Auswerteeinheit erstellt insofern ein vollständiges Protokoll der Messung, das die kombinierte Auswertung der verschiedenen Messdatensätze zur Bildrekonstruktion erlaubt. In einer bevorzugten Ausgestaltung der Erfindung führt sie auf der Basis des Protokolls auch die Rekonstruktion durch und berechnet so die gewünschten tomographischen Bilder.

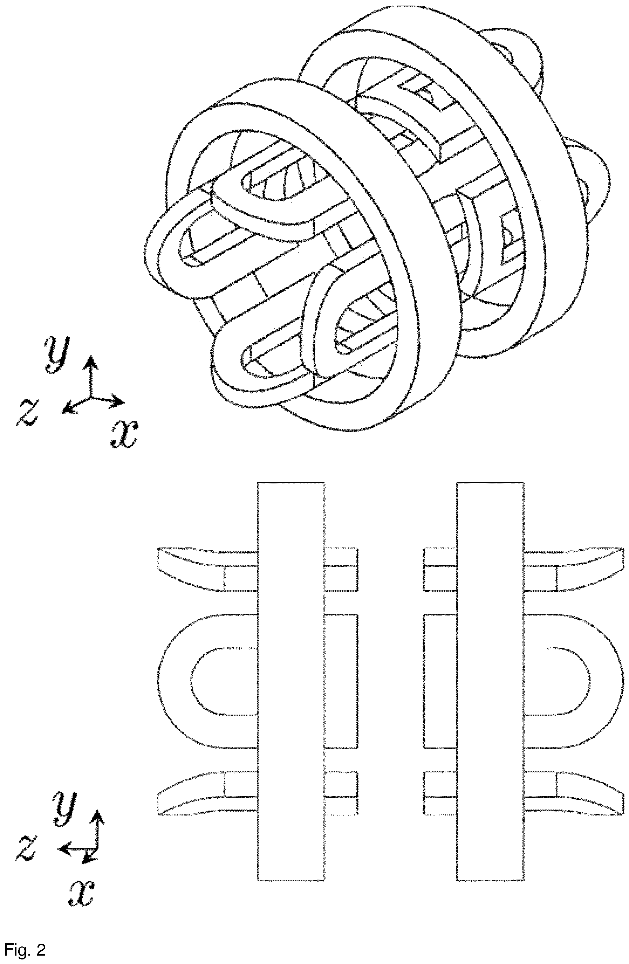

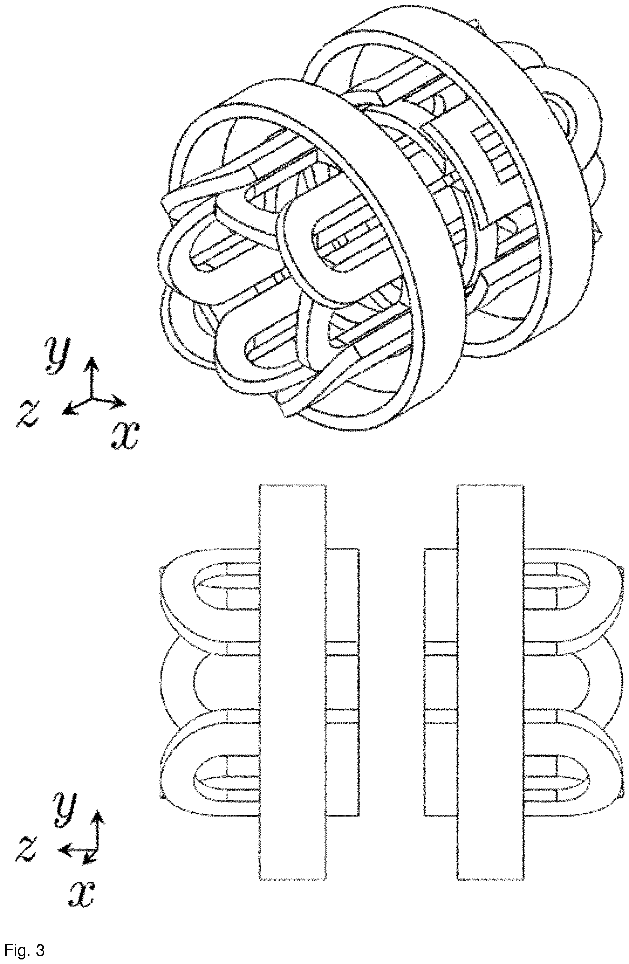

Zur Veranschaulichung der möglichen Ausgestaltung einer MPI-fähigen Spulenanordnung für einen hybriden MPI-CT-Scanner dienen die Figuren. In allen Figuren ist ein kartesisches Dreibein zur Indizierung der Richtungen eingezeichnet. Die Spulenanordnungen der Figuren 2 und 3 sind beispielsweise geeignet zur Verwendung in einer erfindungsgemäßen Vorrichtung, bei der der CT-Scanner-Gantry in der xy-Ebene angeordnet ist. Der CT-Scanner selbst wird nicht dargestellt. Die Durchstrahlungsebene fällt hier stets mit der xy-Ebene zusammen, d.h. der in den Figuren sichtbare Spalt zwischen den Teilen der Spulenanordnungen wird vom Röntgenstrahl durchsetzt. Es befindet sich grundsätzlich außer dem zu durchleuchtenden Patienten kein anderes Hindernis im Röntgenstrahl, insbesondere kein metallisches Spulenstück. Derartige Spulenstücke gäben ansonsten Anlass zu Artefakten in der CT-Rekonstruktion.

Aus Fig. 1 ist ersichtlich, dass eine FFL mit wenigstens zehn Spulen erzeugt werden kann, wobei zwei Spulen parallel zur xy-Ebene ausgerichtet sind. Die übrigen acht schneiden die xy-Ebene. Ein einzelner FFP kann mit einer Anordnung aus drei senkrecht zueinander stehenden Spulenpaaren erzeugt und in einem bestimmten Gebiet innerhalb der Anordnung bewegt werden. Auch hier kann ein Spulenpaar parallel zur xy-Ebene ausgerichtet sein, während die übrigen vier Spulen die xy-Ebene schneiden.

Es ist die erfindungsgemäße Ausgestaltung eines hybriden MPI-CT-Scanners, jene Spulen der MPI-fähigen Anordnung, die die Durchstrahlungsebene des Röntgenstrahls schneiden, durch separierte Partialspulenpaare zu ersetzen.

Als ein separiertes Partialspulenpaar soll in dieser Beschreibung ein Paar apparativ separierter, geschlossener Spulen mit einer vorbestimmten Anordnung zueinander bezeichnet werden. Jede der Partialspulen weist dabei einen konstruktiven und einen destruktiven Spulenabschnitt auf in dem Sinne, dass ein Stromfluss durch den konstruktiven Abschnitt der Erzeugung eines gewünschten Magnetfeldes dient, wohingegen der Stromfluss durch den destruktiven Abschnitt ein unerwünschtes Magnetfeld erzeugt. Die vorbestimmte Anordnung der Partialspulen soll idealerweise nur das gewünschte Magnetfeld generieren und ist daher stets so ausgebildet, dass sich die destruktiven Abschnitte der Partialspulen nah beieinander gegenüberliegen und entgegengesetzt von Strom durchflossen sind. Bevorzugt sind die Partialspulen eines Paares baugleich, und sie werden von gleich großen Strömen durchflossen. Die destruktiven Spulenabschnitte verlaufen vorzugsweise parallel, aber nicht notwendig in einer Ebene, d.h. sie können auch in einer gekrümmten Fläche liegen. Ein anschauliches Beispiel für ein separiertes Partialspulenpaar kann man wie folgt skizzieren:
Eine runde, planare Spule wird entlang ihres Durchmessers aufgetrennt. Jede der beiden Spulenhälften wird sodann - Draht für Draht in gerader Linie - zwischen ihren offenen Enden verbunden, so dass zwei geschlossene Spulen mit je einem halbkreisförmigen - konstruktiven - und einem geraden - destruktiven - Spulenabschnitt gebildet worden sind. Diese aufgrund ihrer Form auch als D-Spulen bezeichneten Partialspulen bilden dann ein separiertes Partialspulenpaar, wenn sie in einer Ebene liegend mit ihren geraden Spulenabschnitten direkt gegenüberliegend angeordnet werden. Lässt man gleich große, in den geraden Abschnitten einander entgegen gerichtete Ströme fließen, löschen sich die Magnetfeldbeiträge dieser destruktiven Abschnitte annähernd aus. Demgegenüber erzeugen die beiden halbkreisförmigen, konstruktiven Spulenabschnitte annähernd dasselbe Magnetfeld wie die ursprüngliche runde, planare Spule.

Die separierten Spulenpaare müssen nicht notwendigerweise Spulenabschnitte in Kreissegmentform aufweisen; sie können beliebig geformt sein, beispielsweise als Rechteckspulen vier gerade Abschnitte aufweisen. Die Spulen eines separierten Spulenpaares müssen nicht planar sein, und auch wenn sie planar sind, müssen sie nicht in einer gemeinsamen Ebene angeordnet sein. Charakteristisches Kennzeichen eines separierten Spulenpaares ist das Vorhandensein eines destruktiven Spulenabschnitts in jeder der beiden Spulen und die Anordnung der Spulen derart, dass die destruktiven Spulenabschnitte in einem vorbestimmten Abstand parallel verlaufen. Dieser Abstand definiert die Breite eines Zwischenraumes zwischen den Spulen, der selbst keinen Spulenabschnitt enthält.

In Fig. 2 ist eine Spulenanordnung zur Erzeugung und Bewegung eines einzelnen FFP dargestellt, bei der die beiden Spulenpaare, die die xy-Ebene schneiden würden, durch je zwei separierte Spulenpaare - hier: verlängerte D-Spulen - ersetzt worden sind. Es sind also insgesamt acht D-Spulen zu sehen, wobei je vier oberhalb und unterhalb der xy-Ebene angeordnet sind. Der gemeinsame Zwischenraum aller separierten Spulenpaare bildet einen Spalt, der die xy-Ebene und eine Umgebung vorbestimmter Breite entlang der z-Achse umfasst, wie gezeigt in Fig. 2 unten. Zu beiden Seiten des Spalts liegen in Fig. 2 baugleiche Spulenanordnungen vor.

Jede der beiden baugleichen Spulenanordnungen kann an eine Seite eines CT-Scanner-Gantry herangeführt und dort justiert werden. Am CT-Scanner selbst sind bauliche Veränderungen nicht zwingend erforderlich. Das Erzeugen und Bewegen eines FFP in der xy-Ebene wird durch die synchrone Ansteuerung und Bestromung beider Spulenanordnungen ermöglicht. Der CT-Röntgenstrahl wird nun nirgends an der Durchleuchtung der Durchstrahlungsebene gehindert.

In Fig. 3 ist eine Spulenanordnung zur Erzeugung einer FFL dargestellt. In Anlehnung an Fig. 1 werden hier acht Spulen benutzt, die die xy-Ebene schneiden würden, wenn sie nicht jeweils durch ein separiertes Spulenpaar ersetzt worden wären. Wie zuvor in Fig. 2 werden auch hier zwei baugleiche Spulenanordnungen gezeigt, die zu beiden Seiten eines CT-Scanner-Gantry anzuordnen und zu justieren sind. Die synchrone Ansteuerung und Bestromung der Gesamtanordnung aus Fig. 3 ist in der Lage, ein Magnetfeld mit FFL in der xy-Ebene zu erzeugen, zu bewegen und/oder um beliebige Winkel zu verdrehen.

Zur Schaffung des hindernisfreien Spalts für den Eintritt des Röntgenlichts werden, wie zuvor beschrieben, zahlreiche Spulen durch separierte Spulenpaare ersetzt. Dieses Konzept setzt auf die weitgehende Auslöschung der Magnetfeldbeiträge aller destruktiven Spulenabschnitte. Diese Spulenabschnitte befinden sich jedoch zwangsläufig in unmittelbarer Nachbarschaft der xy-Ebene, in der die feldfreien Punkte oder Linien gebildet und kontrolliert werden müssen. Sind die separierten Spulenpaare nicht sehr präzise zueinander angeordnet - etwa wenn die Teilspulenanordnungen, die von beiden Seiten an den CT-Scanner herangeführt worden sind, nicht ausreichend justiert sind - können Abweichungen des erzeugten Magnetfeldes von der erwarteten Feldverteilung auftreten. Dies kann auch bei perfekter Justierung geschehen, wenn sich mechanische Schwingungen auf die Spulenanordnung durch Körperschall übertragen. Insbesondere können solche Schwingungen auch durch das fortlaufende Umpolen der Magnetfelder selbst angeregt werden.

Wie bereits zuvor erläutert, ergibt sich auch aus dem komplexeren Aufbau eines hybriden MPI-CT-Scanners die Notwendigkeit einer zusätzlichen Überwachung des tatsächlich erzeugten Magnetfeldes nach Erfindungsschritt e. und der Einbeziehung festgestellter Abweichungen in die nachträgliche Bildrekonstruktion gemäß Erfindungsschritt f.

Mittels der Erfindung ist es nunmehr möglich, das Röntgen-CT-Verfahren und das MPI-Verfahren in einer einzigen Messung zu vereinen und dabei die Röntgenabschwächungskoeffizienten und Partikelkonzentrationen zur selben Zeit in demselben, mit dem Patientenkörper verbundenen Koordinatensystem zu erfassen. Durch die kombinierte Datenerfassung wird eine effizientere und/oder genauere Bildrekonstruktion bereitgestellt, und durch eine gemeinsame Auswertung sind zudem neue Detektionsmöglichkeiten, z.B. für Tumorgewebe oder Kalzifizierungen zu erwarten.

## Patentansprüche

1. Hybrider Scanner zur medizinischen Bildgebung umfassend
• eine Einrichtung zur Erzeugung eines den Patienten durchstrahlenden Röntgenstrahls mit vorgebbarer Strahlrichtung in einer den Patienten durchsetzenden Durchstrahlungsebene und
• einen elektronischen Flächendetektor zur Erfassung der Röntgenbilder,
• wobei der hybride Scanner weiterhin eine Anordnung von Magnetfelderzeugerspulen und eine Einrichtung ausgebildet zur Bestromung der Spulen zur Erzeugung eines magnetischen Gradientenfeldes mit wenigstens einem feldfreien Punkt in der Durchstrahlungsebene umfasst,
• wobei der hybride Scanner weiterhin eine erste Ansteuereinrichtung, die der Einrichtung zur Erzeugung des Röntgenstrahls die Strahlrichtung vorgibt, und
• eine zweite Ansteuereinrichtung, die die Bewegung des wenigstens einen feldfreien Punktes in der Durchstrahlungsebene kontrolliert, umfasst,
• wobei der hybride Scanner weiterhin Detektionsmittel zum Erfassen der magnetischen Antwort der magnetisierbaren Partikel auf die Bewegung des wenigstens einen feldfreien Punktes sowie eine wenigstens mit der ersten und mit der zweiten Ansteuereinrichtung und mit dem Flächendetektor und mit den Detektionsmitteln elektronisch kommunizierende Recheneinheit umfasst,
**dadurch gekennzeichnet, dass**
• die Anordnung von Magnetfelderzeugerspulen wenigstens zum Teil aus separierten Partialspulenpaaren gebildet ist,
• wobei die Durchstrahlungsebene keine der Magnetfelderzeugerspulen schneidet,
• wobei die Partialspulenpaare jeweils ein Paar apparativ separierter, geschlossener Spulen mit einem konstruktiven und einem destruktiven Spulenabschnitt aufweisen,
• wobei die destruktiven Abschnitte der Partialspulen nah beieinander gegenüberliegend sind, wobei die destruktiven Spulenabschnitte parallel verlaufend sind und
• wobei die destruktiven Spulenabschnitte unter entgegengesetzt gleich großem Stromfluss zur gegenseitigen Auslöschung ihrer Magnetfeldbeiträge geeignet sind.

## Claims

1. A hybrid scanner for medical imaging, comprising
• a device for generating an X-ray beam irradiating through the patient, said beam having a predefinable direction of radiation in a radiation plane passing through the patient, and
• an electronic area detector for detecting the X-ray images,
• wherein the hybrid scanner also comprises an assembly of magnetic field generator coils and a device designed to energise the coils in order to generate a magnetic gradient field having at least one field-free point in the radiation plane,
• wherein the hybrid scanner also comprises a first control device, which specifies the radiation direction for the device for generating the X-ray beam, and
• a second control device, which controls the movement of the at least one field-free point in the radiation plane,
• wherein the hybrid scanner also comprises detection means for detecting the magnetic response of the magnetisable particles to the movement of the at least one field-free point, and a computing unit electronically communicating with the first and with the second control device and with the area detector and with the detection means,
**characterised in that**
• the arrangement of magnetic field generator coils is formed at least in part of separated partial coil pairs,
• wherein the radiation plane does not intersect any of the magnetic field generator coils,
• wherein the partial coil pairs each comprise a pair of separated closed coils having a constructive and a destructive coil portion,
• wherein the destructive portions of the partial coils are arranged oppositely, closely to one another, wherein the destructive coil portions run in parallel, and
• wherein the destructive coil portions are suitable for mutual cancellation of their magnetic field contributions under opposed current flow of identical magnitude.

## Revendications

1. Scanner hybride destiné à l'imagerie médicale, comprenant
• un dispositif de génération d'un rayon radiographique traversant le patient et doté d'un sens de rayon prédéfinissable dans un plan de rayonnement traversant le patient et
• un détecteur électronique de surface pour enregistrer les images radiographiques,
• le scanner hybride comprenant en outre un dispositif de bobines de génération de champ magnétique et un dispositif conçu pour alimenter les bobines en courant pour générer un champ de gradient magnétique avec au moins un point sans champ dans le plan de rayonnement,
• le scanner hybride comprenant en outre un premier dispositif de commande qui prescrit au dispositif de génération du rayon radiographique le sens de rayonnement pour générer le rayon radiographique, et
• un second dispositif de commande qui contrôle le mouvement de l'au moins un point sans champ dans le plan de rayonnement,
• le scanner hybride comprenant en outre des moyens de détection pour enregistrer la réponse magnétique des particules magnétisables au mouvement de l'au moins un point sans champ et une unité de calcul communiquant par système électronique au moins avec le premier et avec le second dispositif de commande et avec le détecteur de surface et avec les moyens de détection, **caractérisé en ce que**
• le dispositif de bobines de génération de champ magnétique est constitué au moins en partie de paires de bobines partielles séparées,
• le plan de rayonnement ne coupant aucune des bobines de génération de champ magnétique,
• la paire de bobines partielles présentant respectivement une paire de bobines fermées séparées au niveau appareillage et une section de bobine constructive et une destructive,
• les sections destructives des bobines partielles étant superposées les unes près des autres, les sections destructives de bobines étant orientées parallèlement, et
• les sections destructives de bobines étant aptes, sous un flux opposé de courant d'amplitude égale, aptes à effacer leurs contributions au champ magnétique.
